# EUROPEAN PATENT APPLICATION

(11) **EP 1 938 827 A1**
(43) Date of publication of application: **02.07.2008**
(21) Application number: 06798232.2
(22) Date of filing: 15.09.2006
(51) Int. Cl.: A61K 36/18, A61K 8/97, A61P 9/10, A61P 11/00, A61P 17/02, A61P 17/06, A61P 19/02, A61P 27/02, A61P 27/06, A61P 29/00, A61P 35/00, A61P 35/04, A61P 43/00, A61Q 1/00, A61Q 1/12

(54) **NOVEL INHIBITOR OF VASCULAR ENDOTHELIAL GROWTH FACTOR EXPRESSION**

(30) Priority: 16.09.2005 JP 2005269646; 16.09.2005 JP 2005270179; 26.09.2005 JP 2005277870
(71) Applicant: Shiseido Company, Limited, Chuo-ku Tokyo 104-8010 (JP)
(72) Inventor: HASEGAWA, Kiyotaka, Yokohama-shi Kanagawa 224-8558 (JP); INOMATA, Shinji, Yokohama-shi Kanagawa 224-8558 (JP); UMISHIO, Kenichi, Yokohama-shi Kanagawa 224-8558 (JP)
(74) Representative: Ulmann, Catherine Claire
(86) International application number: PCT/JP2006/318793
(87) International publication number: WO 2007/032561

(57) **Abstract**

The invention provides anti-aging agents, angiogenesis inhibitors and vascular endothelial growth factor (VEGF) expression inhibitors containing one or more galenicals selected from the group consisting of mukurossi peel extract, hazelnut extract, linden extract, coix extract, chlorella extract and tormentilla extract.

## Description

### Technical Field

The present invention relates to anti-aging agents, especially anti-aging agents exhibiting anti-photoaging effects, and to angiogenesis inhibitors or vascular endothelial growth factor (VEGF) expression inhibitors.

### Background Art

Angiogenesis is the phenomenon of new formation of blood vessels. Blood vessels are newly formed by a process in which vascular endothelial cells are released and proliferate during degradation of the vascular basement membrane by the action of proteases in tissues and organs, and then adhere to the extracellular matrix and differentiate to form the vascular lumen. Angiogenesis, in addition to be involved in a normal response in the wound healing process, is also known as a cause or aggravator of various diseases such as cancer (proliferation and metastasis), diabetic retinopathy, neovascular glaucoma, inflammatory skin conditions, psoriasis, rheumatoid arthritis, osteoarthritis, age-related macular degeneration, chronic bronchitis, atherosclerosis and myocardial infarction.

Among the known angiogenic factors, vascular endothelial growth factor (VEGF) is not only involved in angiogenesis, but it also has a variety of other functions in the vascular, blood and clotting systems such as inducing proliferation and maintaining the survival of vascular endothelial cells, promoting vascular permeability, regulating blood pressure, as well as promoting migration of platelets and chemotaxis of macrophages. Compounds that inhibit VEGF expression or activity of vascular endothelial cells are expected to be useful as angiogenesis inhibitors for remedying or preventing numerous above-mentioned diseases such as cancer, diabetic retinopathy and different types of arthritis, or as drugs for prevention or amelioration of skin aging.

It has been reported in recent years that endothelial cells in the dermovascular system also play a part in skin aging (International Patent Publication No. W02003/084302).

The dermovascular system is essential for maintenance of normal skin structure and function. The dermovascular system also plays an important role in controlling hair growth, mollifying skin damage caused by ultraviolet rays and age-related skin aging, in the skin response against irritation and inflammation and in tissue repair. Blood vessels function stationary in normal human skin but are rapidly activated in response to different stimuli, and their reactions are accompanied by vasodilation and angiogenesis, whereby new capillaries are formed from existing vessels. Such changes are dependent on the balance between neovascularization molecules (angiogenesis-promoting factors) and anti-vascularization molecules vascularization molecule (angiogenesis-inhibiting factors). It has been demonstrated that endogenous angiogenesis-inhibiting factors are more predominant than angiogenesis-promoting factors in normal skin.

VEGF is also known to be a vascular permeability factor, but recent research has suggested that it is a major angiogenesis-promoting factor in human skin. In healthy skin, VEGF is secreted in small amounts from keratinocytes and binds to specific receptors on microvascular endothelial cells in the dermis, whereby it ensures survivability of the endothelial cells and helps support the vasculature in the upper network structure. VEGF is very highly expressed in hyperplasic cuticle during inflammation or wound healing, and thus increases vascularization and nutrient supply in the dermis for the cuticle which lacks vessels.

Results of in vivo experiments using transgenic mice overexpressing VEGF or angiogenesis-inhibiting factors in the skin have indicated that regulating the level of these angiogenesis-promoting or angiogenesis-inhibiting factors can notably affect the extent of ultraviolet ray-induced damage and skin wrinkling, as well as skin response to stimulation and skin wound repair (International Patent Publication No. WO2003/084302).

Prolonged irradiation of mice with ultraviolet rays produces extensive wrinkling, but it has been found, surprisingly, that the marked growth of subcutaneous vessels that occurs during this process is accompanied by increased vascular dilation and branching. Prolonged irradiation of mice with ultraviolet rays has also been found to promote expression of vascular endothelial growth factor. In light of these facts, it has become clear that neoformation of vascular endothelial cells is a factor involved in formation of wrinkles by skin injury, or in other words, that it should be possible to prevent formation of wrinkles by inhibiting neoformation of vascular endothelial cells and inducing death of vascular endothelial cells.

### DISCLOSURE OF THE INVENTION

It is an object of the invention to provide novel anti-aging agents, angiogenesis inhibitors or VEGF expression inhibitors.

The present inventors have discovered, after much ardent research, that expression of VEGF is inhibited by certain galenicals, and specifically mukurossi peel extract, hazelnut extract, linden extract, coix extract, chlorella extract and tormentilla extract.

One aspect of the invention, therefore, provides anti-aging agents comprising one or more drugs selected from the group consisting of the galenicals mukurossi peel extract and hazelnut extract. Such anti-aging agents are highly effective for preventing or inhibiting formation of wrinkles, and especially formation of wrinkles by photoaging.

The invention further relates to a method for inhibiting aging, which method includes application of an anti-aging agent as described above to skin. The method may also be a cosmetic method which is not for medical practice aimed at treatment of diseased skin.

Another aspect of the invention provides angiogenesis inhibitors comprising one or more drugs selected from the group consisting of mukurossi peel extract and hazelnut extract. The invention further provides VEGF expression inhibitors comprising one or more drugs selected from the group consisting of mukurossi peel extract and hazelnut extract.

The invention still further provides angiogenesis inhibitors characterized by comprising the galenical linden extract as an active component.

Yet another aspect of the invention provides vascular endothelial cell proliferation factor expression inhibitors characterized by comprising linden extract as an active component.

The invention still further provides vascular endothelial growth factor expression inhibitors characterized by comprising one or more galenical extracts selected from the group consisting of the galenicals coix extract, chlorella extract and tormentilla extract, as active components.

### Brief Description of the Drawings

Fig. 1 shows the results of screening galenicals having VEGF expression inhibiting activity by luciferase assay.
Fig. 2 shows the results of screening galenicals having VEGF expression inhibiting activity by luciferase assay.
Fig. 3 shows the results of screening galenicals having VEGF expression inhibiting activity by luciferase assay.
Fig. 4 shows the effects of mukurossi peel extract and hazelnut extract on VEGF protein expression in normal cells.
Fig. 5 shows the effect of linden extract on VEGF protein expression in normal cells.
Fig. 6 shows the effects of coix extract, chlorella extract and tormentilla extract on VEGF protein expression in normal cells.

### Best Mode for Carrying Out the Invention

The screening of galenicals that inhibit VEGF expression is described in detail in the examples and in Experiment 1 below, but basically it was carried out by allowing different candidate drugs to act on a HaCat cell line having transferred therein a DNA construct comprising luciferase as a reporter gene linked downstream from the VEGF promoter, measuring the luciferase activity, defining VEGF expression inhibition as suppression of luciferase activity, and selecting out as effective drugs those having VEGF expression inhibiting activity. The actual VEGF expression inhibiting effects of drugs selected in this manner were also examined in normal cells.

As a result, it was discovered that mukurossi peel extract, hazelnut extract, linden extract, coix extract, chlorella extract and tormentilla extract significantly inhibit VEGF expression. It was further concluded that mukurossi peel extract and hazelnut extract are effective for suppressing skin aging and for treatment or prevention of diseases associated with angiogenesis, such as cancer, that linden extract is an effective angiogenesis inhibitor and that coix extract, chlorella extract and tormentilla extract are effective VEGF inhibitors.

Mukurossi peel extract: An extract from the peel of Sapondus mukurossi Gaertner (Sapindaceae). It is a natural component widely used in toothpastes, shampoos and soaps.

Hazelnut extract: An extract from the seeds of Guevina avellana Mol (Proteaceae). Edible oil used in cooking is derived from the fruit, and because this oil also prevents skin drying it is used in soaps, packs and massage oils.

Linden extract: An extract from the flower or leaves of linden (Tilia cordata Mill.), a defoliating tree indigenous to southern and central Europe. It is known to have diaphoretic and sedative effects, and is used as a medicinal herb in bath additives. Linden extract also includes the extract from flowers or leaves of Tilia cordata Mill, Tilia platyphyllos Scop. and Tilia europaea Linne (Tiliaceae).

Coix extract: An extract from the episperm-removed seeds of Coix lacryma-jobi Linne var. ma-yuen Stapt (Gramineae). Its major components are amino acids such as glutamic acid, leucine and tyrosine, and coixenolide. It also known to contain abundant natural minerals and exhibit an excellent skin moisture retention effect.

Chlorella extract: An extract obtained from Chlorella vulgaris Chick (Chlorellaceae), a fresh water-growing unicellular plant. It contains an abundance of amino acids and vitamins as major components. It is known to have cell mitosis-inducing and anti-tumor effects.

Tormentilla extract: An extract obtained from the roots of Potentilla tormentilla Schrk (Rosaceae), a plant of the Rosaceae family. It is known to suppress excess oxidation and eliminate active oxygen.

These extracts can all be obtained by ordinary methods, and for example, the plant sources for each of the extracts may be soaked or heated to reflux with an extraction solvent and then filtered and concentrated. The extraction solvent used may be any one ordinarily employed for extraction, and for example, water or organic solvents including alcohols such as methanol, ethanol, propylene glycol, 1,3-butylene glycol or glycerin, water-containing alcohols, chloroform, dichloroethane, carbon tetrachloride, acetone, ethyl acetate, hexane and the like may be used either alone or in combinations. The extract obtained by extraction with the solvent may be used directly, or the concentrated extract may be depleted of impurities by an adsorption process using an ion exchange resin or the like, or it may be subjected to adsorption on a porous polymer (for example, AMBERLITE XAD-2) column and then eluted with methanol or ethanol and concentrated. An extract obtained by a partition method using water/ethyl acetate may also be used.

The anti-aging agents of the invention are highly effective for preventing and inhibiting aging caused by neoformation, growth and proliferation of vascular endothelial cells. Aging caused by neoformation, growth and proliferation of vascular endothelial cells includes photoaging resulting from skin injury or exposure to ultraviolet rays. The galenicals of the invention are especially effective for preventing and reducing photoaging.

The angiogenesis inhibitors or VEGF expression inhibitors of the invention can be used as drugs or cosmetics effective for accelerating wound healing, for treating and preventing conditions associated with neoformation, growth and proliferation of vascular endothelial cells, such as cancer proliferation or metastasis, diabetic retinopathy, neovascular glaucoma, inflammatory skin diseases, psoriasis, rheumatoid arthritis, osteoarthritis, age-related macular degeneration, chronic bronchitis, atherosclerosis, myocardial infarction and the like, and for preventing and ameliorating aging caused by neoformation, growth and proliferation of vascular endothelial cells. Aging caused by neoformation, growth and proliferation of vascular endothelial cells includes photoaging resulting from skin injury or exposure to ultraviolet rays.

Photoaging refers to changes in the appearance and function of skin resulting from repeated exposure to sunlight, primarily. Ultraviolet (UV) light, which is a component of sunlight, and especially intermediate UV (UVB, having a wavelength of 290-320 nm) is the major cause of photoaging. The amount of exposure to UVB necessary to cause photoaging is unknown at the current time. However, normal photoaging is linked to repeated exposure to UVB of a level that produces erythema or sunburn. Clinically, photoaging is identified by skin roughening, wrinkle formation, discoloration, darkening, sag formation, telangiectasia, mole formation, purpura, susceptibility to injury, atrophy, fibrotic depigmentation regions, premalignant tumors and malignant tumors. Photoaging normally occurs in skin that is habitually exposed to sunlight, such as the face, ears, head, neck and hands.

The content of galenicals that inhibit angiogenesis in an anti-aging agent, angiogenesis inhibitor or VEGF expression inhibitor according to the invention is 0.0001-20.0 wt% and preferably 0.0001-10.0 wt% as dry weight with respect to the total agent.

An anti-aging agent, angiogenesis inhibitor or VEGF expression inhibitor of the invention may contain, in addition to the aforementioned essential components, various components used in ordinary external skin cosmetics and drugs, depending on the purpose, and examples of such components include skin whiteners, humectants, antioxidants, oil components, ultraviolet absorbers, surfactants, thickeners, alcohols, powder constituents, pigments, aqueous components, water and various skin nutrients, as well as components commonly used in foods and drugs such as excipients, moistureproof agents, antiseptic agents, reinforcers, thickeners, emulsifying agents, antioxidants, sweeteners, acidulants, seasonings, coloring agents, aromatics and the like, as appropriate for the need.

In addition, there may also be added appropriate amounts of metal sequestering agents such as disodium edetate, trisodium edetate, sodium citrate, sodium polyphosphate, sodium metaphosphate or gluconic acid, caffeine, tannin, verapamil, tranexamic acid or their derivatives, licorice extract, drug agents such as glabridin, Chinese quince fruit hot water extract, galenicals, tocopherol acetate, glycyrrhizic acid and their derivatives or salts, skin whiteners such as vitamin C, magnesium ascorbate phosphate, glucoside ascorbate, arbutin or kojic acid, saccharides such as glucose, fructose, mannose, sucrose or trehalose, and vitamin A derivatives such as retinoic acid, retinol, retinol acetate or retinol palmitate.

The administration form for an anti-aging agent of the invention is not particularly restricted and may be any type of external preparation conventionally used for skin such as an ointment, cream, emulsion, lotion, pack, bath additive or the like.

The anti-aging agents of the invention can be applied to skin for use in cosmetic methods to prevent or ameliorate aging. The method and dosage for an anti-aging agent of the invention used in a cosmetic method is not particularly restricted and may be appropriately established depending on the formulation or the condition of skin wrinkles to be treated, but typically the optimal dose, e.g. from 0.1 ml to 1 ml per cm², is rubbed directly onto the skin or absorbed in gauze and applied onto the skin, several times, for example, 1 to 5 times per day.

The dosage, method of administration and dosage form of an angiogenesis inhibitor or VEGF expression inhibitor of the invention may be appropriately established according to the purpose of use. For example, the dosage form of an angiogenesis inhibitor or VEGF expression inhibitor of the invention may be oral, parenteral or external. As examples of dosage forms there may be mentioned oral administration forms such as tablets, powders, capsules, granules, extract agents and syrups, parenteral administration forms such as injections, drops and suppositories, and external preparations such as ointments, creams, emulsions, lotions, packs and bath additives.

### Examples

The present invention will now be explained in greater detail by examples. However, the invention is in no way limited by the examples. The contents are expressed as weight percentages.

### Experiment 1

### Screening of galenicals with VEGF expression inhibiting activity

The cell line used was HaCaT cells possessing a stably transferred DNA construct obtained by linking a luciferase-coding gene as a reporter gene downstream from the VEGF promoter. The cell line was suspended in DMEM (Invitrogen) (containing 10% FBS (ICN)) and 400 µg/ml of G418 (Promega)) and then seeded onto a 24-well plate at 4 × 10⁴ cells per well and cultured at 5% CO₂, 37°C. After approximately 24 hours, different dried plant extracts dissolved in DMSO (Wako Pure Chemical Industries, Ltd.) were added as candidate drugs to final concentrations of 10⁻⁴, 10⁻³, 10⁻²% with respect to the culture solution, and culturing was continued for 24 hours. After removal of the culture supernatants, the cells were rinsed with PBS and a Luciferase Assay System (Promega) was used to measure the luciferase activity.

For measurement of the luciferase activity of the cells, Hoechst 33342 (Sigma) was used at a final concentration of 10 µg/ml, to determine the amount of DNA per well. This was taken as an index of the number of cells per well, and the luciferase activity per cell was calculated.

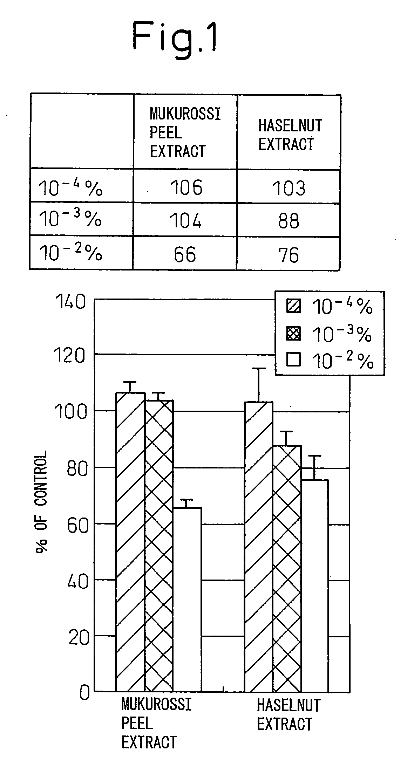

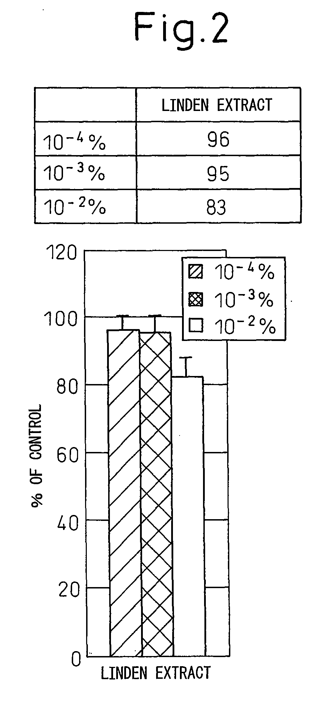

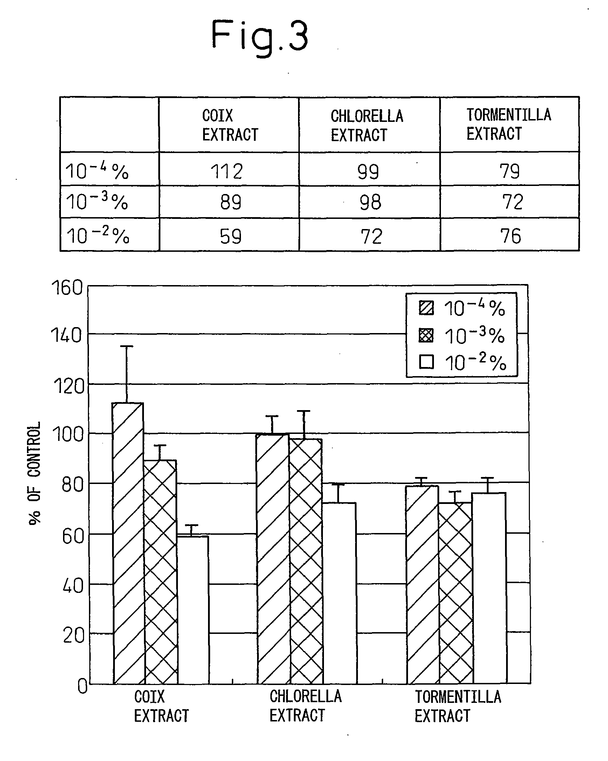

As a result, of the different plant extracts tested, mukurossi peel extract, hazelnut extract, linden extract, coix extract, chlorella extract and tormentilla extract (Maruzen Pharmaceuticals Co., Ltd.) exhibited concentration-dependent inhibition of VEGF expression. The results are shown in Figs. 1, 2 and 3. As a control, DMSO alone was used instead of the candidate drug. Experiment 2

### Quantitation of VEGF protein in normal cells

The galenicals mukurossi peel extract, hazelnut extract, linden extract, coix extract, chlorella extract and tormentilla extract, which were found to exhibit inhibiting effects on promoter activity in the luciferase assay of Experiment 1, were then examined for actual VEGF protein expression inhibiting effects in normal cells.

Normal human keratinocytes (Kurabo Industries, Ltd.) were suspended in KGM medium (Kurabo Industries, Ltd.), and then seeded onto a 24-well plate at 4 × 10⁴ cells per well and cultured at 5% CO₂, 37°C. After approximately 24 hours, a solution of dried mukurossi peel extract, hazelnut extract, linden extract, coix extract, chlorella extract or tormentilla extract in DMSO (Wako Pure Chemical Industries, Ltd.) was added to final concentrations of 10⁻³, 3.3 × 10⁻³ and 10⁻²% with respect to the culture solution, and culturing was continued for 24 hours. The VEGF protein in the culture supernatant was measured by ELISA (R&D Systems).

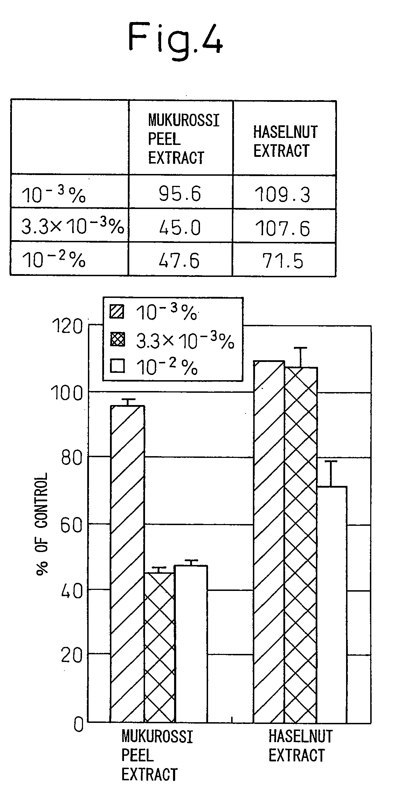

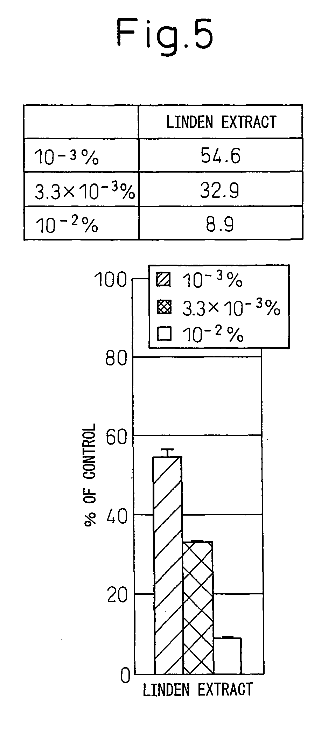

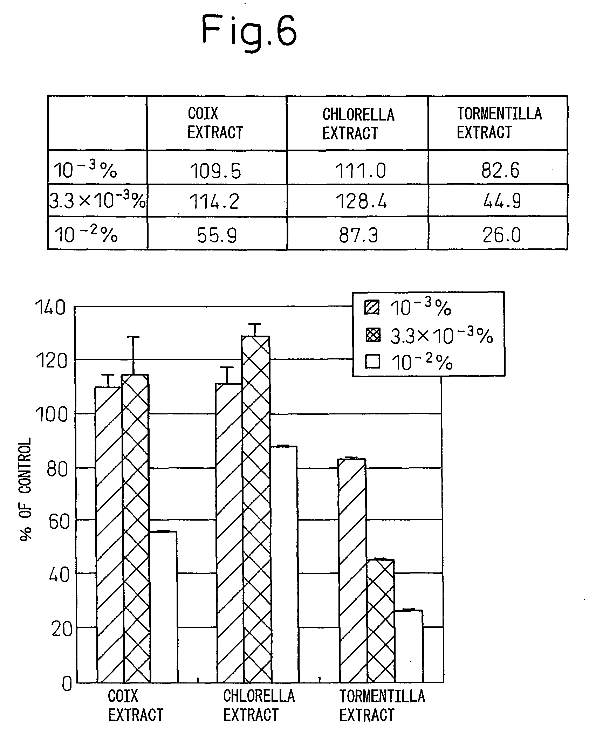

As a result, mukurossi peel extract, hazelnut extract, linden extract, coix extract, chlorella extract and tormentilla extract were confirmed to inhibit VEGF expression in a concentration-dependent manner. The results are shown in Figs. 4, 5 and 6. As a control, DMSO alone was used instead of the candidate drug.

### (Example 1) Cream

### (Ingredients) (wt%)

(1) Stearic acid: 5.0
(2) Stearyl alcohol: 4.0
(3) Isopropyl myristate: 18.0
(4) Glycerin monostearate: 3.0
(5) Propylene glycol: 10.0
(6) Mukurossi peel extract: 0.01
(7) Caustic potash: 0.2
(8) Sodium hydrogensulfite: 0.01
(9) Preservative: q.s.
(10) Aromatic: q.s.
(11) Ion-exchanged water: remainder

### (Preparation Method)

Components (5)-(7) are added to and dissolved in (11), and the mixture is heated and held at 70°C (aqueous phase). Separately, (1)-(4) and (8)-(10) are combined, heated to melting and held at 70°C (oily phase). The oily phase is slowly added to the aqueous phase, and reaction is conducted a short while after completing the addition, while maintaining the temperature. The mixture is then homogeneously emulsified in a homomixer and cooled to 30°C while stirring.

### (Example 2) Cream

### (Ingredients) (wt%)

(1) Stearic acid: 2.0
(2) Stearyl alcohol: 7.0
(3) Hydrogenated lanolin: 2.0
(4) Squalane: 5.0
(5) 2-Octyldodecyl alcohol: 6.0
(6) Polyoxyethylene(25 mol)cetyl alcohol ether: 3.0
(7) Glycerin monostearate: 2.0
(8) Propylene glycol: 5.0
(9) Hazelnut extract: 0.05
(10) Sodium hydrogensulfite: 0.03
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is added to the aqueous phase and pre-emulsified, and after homogeneously emulsifying in a homomixer, the mixture is cooled to 30°C while mixing.

### (Example 3) Cream

### (Ingredients) (wt%)

(1) Solid paraffin: 5.0
(2) Beeswax: 10.0
(3) Vaseline: 15.0
(4) Liquid paraffin: 41.0
(5) Glycerin monostearate: 2.0
(6) Polyoxyethylene(20 mol) sorbitan monolaurate: 2.0
(7) Soap powder: 0.1
(8) Borax: 0.2
(9) Mukurossi peel extract: 0.05
(10) Hazelnut extract: 0.05
(11) Sodium hydrogensulfite: 0.03
(12) Ethylparaben: 0.3
(13) Aromatic: q.s.
(14) Ion-exchanged water: remainder

### (Preparation Method)

Components (7) and (8) are added to (14), and the mixture is heated and held at 70°C (aqueous phase). Separately, (1)-(6) and (9)-(14) are combined, heated to melting and held at 70°C (oily phase). The oily phase is slowly added to the aqueous phase while stirring, for reaction. Upon completion of the reaction, the mixture is homogeneously emulsified in a homomixer, and then thoroughly stirred while cooling to 30°C.

### (Example 4) Emulsion

### (Ingredients) (wt%)

(1) Stearic acid: 2.5
(2) Cetyl alcohol: 1.5
(3) Vaseline: 5.0
(4) Liquid paraffin: 10.0
(5) Polyoxyethylene(10 mol) monooleate: 2.0
(6) Polyethylene glycol 1500: 3.0
(7) Triethanolamine: 1.0
(8) Carboxyvinyl polymer: 0.05
   (Carbopol 941, B.F. Goodrich)
(9) Mukurossi peel extract: 0.01
(10) Sodium hydrogensulfite: 0.01
(11) Ethylparaben: 0.3
(12) Aromatic: q.s.
(13) Ion-exchanged water: remainder

### (Preparation Method)

Component (8) is dissolved in a small amount of (13) (phase A). Components (6) and (7) are added to and dissolved in the remainder of (13), and the mixture is heated and held at 70°C (aqueous phase). Separately, (1)-(5) and (9)-(12) are combined, heated to melting and held at 70°C (oily phase). The oily phase is added to the aqueous phase and pre-emulsified, and after adding phase A and homogeneously emulsifying in a homomixer, the mixture is cooled to 30°C while mixing.

### (Example 5) Emulsion

### (Ingredients) (wt%)

(1) Microcrystalline wax: 1.0
(2) Beeswax: 2.0
(3) Lanolin: 20.0
(4) Liquid paraffin: 10.0
(5) Squalane: 5.0
(6) Sorbitan sesquioleate: 4.0
(7) Polyoxyethylene(20 mol) sorbitan monooleate: 1.0
(8) Propylene glycol: 7.0
(9) Hazelnut extract: 1.0
(10) Sodium hydrogensulfite: 0.01
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is stirred while the aqueous phase is slowly added thereto, and the mixture is homogeneously emulsified in a homomixer. The emulsion is then cooled to 30°C while stirring.

### (Example 6) Jelly

### (Ingredients) (wt%)

(1) 95% Ethyl alcohol: 10.0
(2) Dipropylene glycol: 15.0
(3) Polyoxyethylene(50 mol) oleyl alcohol ether: 2.0
(4) Carboxyvinyl polymer: 1.0
   (Carbopol 940, B.F. Goodrich)
(5) Caustic soda: 0.15
(6) L-Arginine: 0.1
(7) Mukurossi peel extract: 7.0
(8) Sodium 2-hydroxy-4-methoxybenzophenonesulfonate: 0.05
(9) Ethylenediaminetetraacetate·3Na·2H₂O: 0.05
(10) Methylparaben: 0.2
(11) Aromatic: q.s.
(12) Ion-exchanged water: remainder

### (Preparation Method)

Component (4) is uniformly dissolved in (12) (aqueous phase). Components (7) and (3) are dissolved in (1), and this is added to the aqueous phase. After then adding (2) and (8)-(11) thereto, the mixture is neutralized and thickened with (5) and (6).

### (Example 7) Essence

### (Ingredients) (wt%)

(Phase A)
   Ethyl alcohol (95%): 10.0
   Polyoxyethylene(20 mol) octyldodecanol: 1.0
   Pantothenyl ethyl ether: 0.1
   Hazelnut extract: 1.5
   Methylparaben: 0.15
(Phase B)
   Potassium hydroxide: 0.1
(Phase C)
   Glycerin: 5.0
   Dipropylene glycol: 10.0
   Sodium hydrogen sulfite: 0.03
   Carboxyvinyl polymer: 0.2
   (Carbopol 940, B.F. Goodrich)
   Purified water: remainder

### (Preparation Method)

Phase A and phase C are each uniformly dissolved, and then phase A is added to phase C for solubilization. After then adding phase B, the mixture is packed into a container.

### (Example 8) Pack

### (Ingredients) (wt%)

(Phase A)
   Dipropylene glycol: 5.0
   Polyoxyethylene(60 mol) hydrogenated castor oil: 5.0
(Phase B)
   Mukurossi peel extract: 0.01
   Olive oil: 5.0
   Tocopherol acetate: 0.2
   Ethylparaben: 0.2
   Aromatic: 0.2
(Phase C)
   Sodium hydrogen sulfite: 0.03
   Polyvinyl alcohol (saponification degree: 90,
   polymerization degree: 2000): 13.0
   Ethanol: 7.0
   Purified water: remainder

### (Preparation Method)

Phase A, phase B and phase C are each uniformly dissolved, and phase B is added to phase A for solubilization. After adding this to phase C, the mixture is packed into a container.

### (Example 9) Solid foundation

### (Ingredients) (wt%)

(1) Talc: 43.1
(2) Kaolin: 15.0
(3) Sericite: 10.0
(4) Zinc flower: 7.0
(5) Titanium dioxide: 3.8
(6) Yellow iron oxide: 2.9
(7) Black iron oxide: 0.2
(8) Squalane: 8.0
(9) Isostearic acid: 4.0
(10) POE sorbitan monooleate: 3.0
(11) Isocetyl octanoate: 2.0
(12) Hazelnut extract: 1.0
(13) Preservative: q.s.
(14) Aromatic: q.s.

### (Preparation Method)

The powder constituents of (1)-(7) are thoroughly mixed with a blender, the oily components (8)-(11) and components (12), (13) and (14) are added and thoroughly kneaded therewith, and the mixture is then packed into a container and shaped.

### (Example 10) Emulsified foundation (cream type)

### (Ingredients) (wt%)

(Powder portion)
   Titanium dioxide: 10.3
   Sericite: 5.4
   Kaolin: 3.0
   Yellow iron oxide: 0.8
   Red iron oxide: 0.3
   Black iron oxide: 0.2
(Oily phase)
   Decamethylcyclopentasiloxane: 11.5
   Liquid paraffin: 4.5
   Polyoxyethylene-modified dimethylpolysiloxane: 4.0
(Aqueous phase)
   Purified water: 51.0
   1,3-Butylene glycol: 4.5
   Mukurossi peel extract: 1.5
   Sorbitan sesquioleate: 3.0
   Preservative: q.s.
   Aromatic: q.s.

### (Preparation Method)

After heating and stirring the aqueous phase, the thoroughly mixed and pulverized powder portion is added and the mixture is treated in a homomixer. The heated and mixed oily phase is added and treated in a homomixer, after which the aromatic is added while stirring and the mixture is cooled to room temperature.

### (Example 11) Cream

### (Ingredients) (wt%)

(1) Stearic acid: 5.0
(2) Stearyl alcohol: 4.0
(3) Isopropyl myristate: 18.0
(4) Glycerin monostearate: 3.0
(5) Propylene glycol: 10.0
(6) Hazelnut extract: 0.01
(7) Caustic potash: 0.2
(8) Sodium hydrogensulfite: 0.01
(9) Preservative: q.s.
(10) Aromatic: q.s.
(11) Ion-exchanged water: remainder

### (Preparation Method)

Components (5)-(7) are added to and dissolved in (11), and the mixture is heated and held at 70°C (aqueous phase). Separately, (1)-(4) and (8)-(10) are combined, heated to melting and held at 70°C (oily phase). The oily phase is slowly added to the aqueous phase, and reaction is conducted a short while after completing the addition, while maintaining the temperature. The mixture is then homogeneously emulsified in a homomixer and cooled to 30°C while stirring.

### (Example 12) Cream

### (Ingredients) (wt%)

(1) Stearic acid: 2.0
(2) Stearyl alcohol: 7.0
(3) Hydrogenated lanolin: 2.0
(4) Squalane: 5.0
(5) 2-Octyldodecyl alcohol: 6.0
(6) Polyoxyethylene(25 mol)cetyl alcohol ether: 3.0
(7) Glycerin monostearate: 2.0
(8) Propylene glycol: 5.0
(9) Mukurossi peel extract: 0.05
(10) Sodium hydrogensulfite: 0.03
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is added to the aqueous phase and pre-emulsified, and after homogeneously emulsifying in a homomixer, the mixture is cooled to 30°C while mixing.

### (Example 13) Cream

### (Ingredients) (wt%)

(1) Solid paraffin: 5.0
(2) Beeswax: 10.0
(3) Vaseline: 15.0
(4) Liquid paraffin: 41.0
(5) Glycerin monostearate: 2.0
(6) Polyoxyethylene(20 mol) sorbitan monolaurate: 2.0
(7) Soap powder: 0.1
(8) Borax: 0.2
(9) Hazelnut extract: 0.05
(10) Sodium hydrogensulfite: 0.03
(11) Ethylparaben: 0.3
(12) Aromatic: q.s.
(13) Ion-exchanged water: remainder

### (Preparation Method)

Components (7) and (8) are added to (13), and the mixture is heated and held at 70°C (aqueous phase) . Separately, (1)-(6) and (9)-(13) are combined, heated to melting and held at 70°C (oily phase). The oily phase is slowly added to the aqueous phase while stirring, for reaction. Upon completion of the reaction, the mixture is homogeneously emulsified in a homomixer, and then thoroughly stirred while cooling to 30°C.

### (Example 14) Emulsion

### (Ingredients) (wt%)

(1) Stearic acid: 2.5
(2) Cetyl alcohol: 1.5
(3) Vaseline: 5.0
(4) Liquid paraffin: 10.0
(5) Polyoxyethylene(10 mol) monooleate: 2.0
(6) Polyethylene glycol 1500: 3.0
(7) Triethanolamine: 1.0
(8) Carboxyvinyl polymer: 0.05
   (Carbopol 941, B.F. Goodrich)
(9) Mukurossi peel extract: 0.01
(10) Sodium hydrogensulfite: 0.01
(11) Ethylparaben: 0.3
(12) Aromatic: q.s.
(13) Ion-exchanged water: remainder

### (Preparation Method)

Component (8) is dissolved in a small amount of (13) (phase A). Components (6) and (7) are added to and dissolved in the remainder of (13), and the mixture is heated and held at 70°C (aqueous phase). Separately, (1)-(5) and (9)-(12) are combined, heated to melting and held at 70°C (oily phase). The oily phase is added to the aqueous phase and pre-emulsified, and after adding phase A and homogeneously emulsifying in a homomixer, the mixture is cooled to 30°C while mixing.

### (Example 15) Emulsion

### (Ingredients) (wt%)

(1) Microcrystalline wax: 1.0
(2) Beeswax: 2.0
(3) Lanolin: 20.0
(4) Liquid paraffin: 10.0
(5) Squalane: 5.0
(6) Sorbitan sesquioleate: 4.0
(7) Polyoxyethylene(20 mol) sorbitan monooleate: 1.0
(8) Propylene glycol: 7.0
(9) Hazelnut extract: 1.0
(10) Sodium hydrogensulfite: 0.01
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is stirred while the aqueous phase is slowly added thereto, and the mixture is homogeneously emulsified in a homomixer. The emulsion is then cooled to 30°C while stirring.

### (Example 16) Jelly

### (Ingredients) (wt%)

(1) 95% Ethyl alcohol: 10.0
(2) Dipropylene glycol: 15.0
(3) Polyoxyethylene(50 mol) oleyl alcohol ether: 2.0
(4) Carboxyvinyl polymer: 1.0
   (Carbopol 940, B.F. Goodrich)
(5) Caustic soda: 0.15
(6) L-Arginine: 0.1
(7) Mukurossi peel extract: 7.0
(8) Sodium 2-hydroxy-4-methoxybenzophenonesulfonate: 0.05
(9) Ethylenediaminetetraacetate·3Na·2H₂O: 0.05
(10) Methylparaben: 0.2
(11) Aromatic: q.s.
(12) Ion-exchanged water: remainder

### (Preparation Method)

Component (4) is uniformly dissolved in (12) (aqueous phase). Components (7) and (3) are dissolved in (1), and this is added to the aqueous phase. After then adding (2) and (8)-(11) thereto, the mixture is neutralized and thickened with (5) and (6).

### (Example 17) Essence

### (Ingredients) (wt%)

(Phase A)
   Ethyl alcohol (95%): 10.0
   Polyoxyethylene(20 mol) octyldodecanol: 1.0
   Pantothenyl ethyl ether: 0.1
   Hazelnut extract: 1.5
   Methylparaben: 0.15
(Phase B)
   Potassium hydroxide: 0.1
(Phase C)
   Glycerin: 5.0
   Dipropylene glycol: 10.0
   Sodium hydrogen sulfite: 0.03
   Carboxyvinyl polymer: 0.2
   (Carbopol 940, B.F. Goodrich)
   Purified water: remainder

### (Preparation Method)

Phase A and phase C are each uniformly dissolved, and then phase A is added to phase C for solubilization. After then adding phase B, the mixture is packed into a container.

### (Example 18) Pack

### (Ingredients) (wt%)

(Phase A)
   Dipropylene glycol: 5.0
   Polyoxyethylene(60 mol) hydrogenated castor oil: 5.0
(Phase B)
   Mukurossi peel extract: 0.01
   Olive oil: 5.0
   Tocopherol acetate: 0.2
   Ethylparaben: 0.2
   Aromatic: 0.2
(Phase C)
   Sodium hydrogen sulfite: 0.03
   Polyvinyl alcohol (saponification degree: 90,
   polymerization degree: 2000): 13.0
   Ethanol: 7.0
   Purified water: remainder

### (Preparation Method)

Phase A, phase B and phase C are each uniformly dissolved, and phase B is added to phase A for solubilization. After adding this to phase C, the mixture is packed into a container.

### (Example 19) Solid foundation

### (Ingredients) (wt%)

(1) Talc: 43.1
(2) Kaolin: 15.0
(3) Sericite: 10.0
(4) Zinc flower: 7.0
(5) Titanium dioxide: 3.8
(6) Yellow iron oxide: 2.9
(7) Black iron oxide: 0.2
(8) Squalane: 8.0
(9) Isostearic acid: 4.0
(10) POE sorbitan monooleate: 3.0
(11) Isocetyl octanoate: 2.0
(12) Hazelnut extract: 1.0
(13) Preservative: q.s.
(14) Aromatic: q.s.

### (Preparation Method)

The powder constituents of (1)-(7) are thoroughly mixed with a blender, the oily components (8)-(11) and components (12), (13) and (14) are added and thoroughly kneaded therewith, and the mixture is then packed into a container and shaped.

### (Example 20) Emulsified foundation (cream type)

### (Ingredients) (wt%)

(Powder portion)
   Titanium dioxide: 10.3
   Sericite: 5.4
   Kaolin: 3.0
   Yellow iron oxide: 0.8
   Red iron oxide: 0.3
   Black iron oxide: 0.2
(Oily phase)
   Decamethylcyclopentasiloxane: 11.5
   Liquid paraffin: 4.5
   Polyoxyethylene-modified dimethylpolysiloxane: 4.0
(Aqueous phase)
   Purified water: 51.0
   1,3-Butylene glycol: 4.5
   Mukurossi peel extract: 1.5
   Sorbitan sesquioleate: 3.0
   Preservative: q.s.
   Aromatic: q.s.

### (Preparation Method)

After heating and stirring the aqueous phase, the thoroughly mixed and pulverized powder portion is added and the mixture is treated in a homomixer. The heated and mixed oily phase is added and treated in a homomixer, after which the aromatic is added while stirring and the mixture is cooled to room temperature.

### (Example 21) Injection

Mukurossi peel extract: 100 mg
Maltosyl-β-cyclodextrin: 726 mg
Sodium hydroxide: 33.3 µg
Distilled water for injection: To 5.0 ml

100 mg of mukurossi peel extract is added to distilled water for injection dissolving the 726 mg of maltosyl-β-cyclodextrin. An aqueous solution of sodium hydroxide (33.3 µg) is then added to a total volume of 5 ml, and the mixture is filtered. After packing the mixture into a vial, it is freeze-dried to obtain an injection.

### (Example 22) Tablets

Hazelnut extract: 1 g
Polyethylene glycol 6000: 10 g
Sodium lauryl sulfate: 1.5 g
Corn starch: 3 g
Lactose: 25 g
Magnesium stearate: 0.5 g

### (Preparation Method)

The components listed above are weighed out. Polyethylene glycol 6000 is heated to 70-80°C, and after adding hazelnut extract, sodium lauryl sulfate, corn starch and lactose, the mixture is cooled. The solidified mixture is granulated in a grinder to obtain granules. After mixing the granules with magnesium stearate, they are compacted and tableted into 250 mg tablets.

### (Example 23) Tablets

Mukurossi peel extract: 3 g
Lactose: 55 g
Potato starch: 12 g
Polyvinyl alcohol: 1.5 g
Magnesium stearate: 1.5 g

### (Preparation Method)

The components listed above are weighed out. Mukurossi peel extract, lactose and potato starch are uniformly mixed. An aqueous solution of polyvinyl alcohol is added to the mixture, and granules are prepared by wet granulation. After drying the granules and mixing with magnesium stearate, they are compacted and tableted into 200 mg tablets.

### (Example 24) Capsules

Hazelnut extract: 1 g
Lactose: 25 g
Corn starch: 5 g
Microcrystalline cellulose: 9.5 g
Magnesium stearate: 0.5 g

### (Preparation Method)

The components listed above are weighed out. The four components other than magnesium stearate are uniformly mixed. After adding magnesium stearate, mixing is continued for several minutes. The mixture is packed into 200 mg hard capsules.

### (Example 25) Powder

Mukurossi peel extract: 2 g
Lactose: 79 g
Magnesium stearate: 1 g
(Preparation Method)

The components listed above are weighed out. All of the components are uniformly mixed to form a powder.

### (Example 26) Suppository

Hazelnut extract: 1 g
Polyethylene glycol 1500: 18 g
Polyethylene glycol 4000: 72 g

### (Preparation Method)

A 1 g rectal suppository is prepared by a melting method.

### (Example 27) Injection

Hazelnut extract: 0.1 g
Sodium chloride: 0.9 g
Sodium hydroxide: q.s.
Water for injection: 100 mL

### (Preparation Method)

The components listed above are weighed out. The three components are dissolved in the water for injection, filtered and sterilized and then dispensed at 5 mL into a 10 mL ampule and heat sealed as an injection.

### (Example 28) Cream

### (Ingredients) (wt%)

(1) Stearic acid: 5.0
(2) Stearyl alcohol: 4.0
(3) Isopropyl myristate: 18.0
(4) Glycerin monostearate: 3.0
(5) Propylene glycol: 10.0
(6) Linden extract: 0.01
(7) Caustic potash: 0.2
(8) Sodium hydrogensulfite: 0.01
(9) Preservative: q.s.
(10) Aromatic: q.s.
(11) Ion-exchanged water: remainder

### (Preparation Method)

Components (5)-(7) are added to and dissolved in (11), and the mixture is heated and held at 70°C (aqueous phase). Separately, (1)-(4) and (8)-(10) are combined, heated to melting and held at 70°C (oily phase). The oily phase is slowly added to the aqueous phase, and reaction is conducted a short while after completing the addition, while maintaining the temperature. The mixture is then homogeneously emulsified in a homomixer and cooled to 30°C while stirring.

### (Example 29) Cream

### (Ingredients) (wt%)

(1) Stearic acid: 2.0
(2) Stearyl alcohol: 7.0
(3) Hydrogenated lanolin: 2.0
(4) Squalane: 5.0
(5) 2-Octyldodecyl alcohol: 6.0
(6) Polyoxyethylene(25 mol)cetyl alcohol ether: 3.0
(7) Glycerin monostearate: 2.0
(8) Propylene glycol: 5.0
(9) Linden extract: 0.05
(10) Sodium hydrogensulfite: 0.03
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is added to the aqueous phase and pre-emulsified, and after homogeneously emulsifying in a homomixer, the mixture is cooled to 30°C while mixing.

### (Example 30) Cream

### (Ingredients) (wt%)

(1) Solid paraffin: 5.0
(2) Beeswax: 10.0
(3) Vaseline: 15.0
(4) Liquid paraffin: 41.0
(5) Glycerin monostearate: 2.0
(6) Polyoxyethylene(20 mol) sorbitan monolaurate: 2.0
(7) Soap powder: 0.1
(8) Borax: 0.2
(9) Linden extract: 0.05
(10) Sodium hydrogensulfite: 0.03
(11) Ethylparaben: 0.3
(12) Aromatic: q.s.
(13) Ion-exchanged water: remainder

### (Preparation Method)

Components (7) and (8) are added to (13), and the mixture is heated and held at 70°C (aqueous phase). Separately, (1)-(6) and (9)-(13) are combined, heated to melting and held at 70°C (oily phase). The oily phase is slowly added to the aqueous phase while stirring, for reaction. Upon completion of the reaction, the mixture is homogeneously emulsified in a homomixer, and then thoroughly stirred while cooling to 30°C.

### (Example 31) Emulsion

### (Ingredients) (wt%)

(1) Stearic acid: 2.5
(2) Cetyl alcohol: 1.5
(3) Vaseline: 5.0
(4) Liquid paraffin: 10.0
(5) Polyoxyethylene(10 mol) monooleate: 2.0
(6) Polyethylene glycol 1500: 3.0
(7) Triethanolamine: 1.0
(8) Carboxyvinyl polymer: 0.05
   (Carbopol 941, B.F. Goodrich)
(9) Linden extract: 0.01
(10) Sodium hydrogensulfite: 0.01
(11) Ethylparaben: 0.3
(12) Aromatic: q.s.
(13) Ion-exchanged water: remainder

### (Preparation Method)

Component (8) is dissolved in a small amount of (13) (phase A). Components (6) and (7) are added to and dissolved in the remainder of (13), and the mixture is heated and held at 70°C (aqueous phase). Separately, (1)-(5) and (9)-(12) are combined, heated to melting and held at 70°C (oily phase). The oily phase is added to the aqueous phase and pre-emulsified, and after adding phase A and homogeneously emulsifying in a homomixer, the mixture is cooled to 30°C while mixing.

### (Example 32) Emulsion

### (Ingredients) (wt%)

(1) Microcrystalline wax: 1.0
(2) Beeswax: 2.0
(3) Lanolin: 20.0
(4) Liquid paraffin: 10.0
(5) Squalane: 5.0
(6) Sorbitan sesquioleate: 4.0
(7) Polyoxyethylene(20 mol) sorbitan monooleate: 1.0
(8) Propylene glycol: 7.0
(9) Linden extract: 1.0
(10) Sodium hydrogensulfite: 0.01
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is stirred while the aqueous phase is slowly added thereto, and the mixture is homogeneously emulsified in a homomixer. The emulsion is then cooled to 30°C while stirring.

### (Example 33) Jelly

### (Ingredients) (wt%)

(1) 95% Ethyl alcohol: 10.0
(2) Dipropylene glycol: 15.0
(3) Polyoxyethylene(50 mol) oleyl alcohol ether: 2.0
(4) Carboxyvinyl polymer: 1.0
   (Carbopol 940, B.F. Goodrich)
(5) Caustic soda: 0.15
(6) L-Arginine: 0.1
(7) Linden extract: 7.0
(8) Sodium 2-hydroxy-4-methoxybenzophenonesulfonate: 0.05
(9) Ethylenediaminetetraacetate·3Na·2H₂O: 0.05
(10) Methylparaben: 0.2
(11) Aromatic: q.s.
(12) Ion-exchanged water: remainder

### (Preparation Method)

Component (4) is uniformly dissolved in (12) (aqueous phase). Components (7) and (3) are dissolved in (1), and this is added to the aqueous phase. After then adding (2) and (8)-(11) thereto, the mixture is neutralized and thickened with (5) and (6).

### (Example 34) Essence

### (Ingredients) (wt%)

(Phase A)
   Ethyl alcohol (95%): 10.0
   Polyoxyethylene(20 mol) octyldodecanol: 1.0
   Pantothenyl ethyl ether: 0.1
   Linden extract: 1.5
   Methylparaben: 0.15
(Phase B)
   Potassium hydroxide: 0.1
(Phase C)
   Glycerin: 5.0
   Dipropylene glycol: 10.0
   Sodium hydrogen sulfite: 0.03
   Carboxyvinyl polymer: 0.2
   (Carbopol 940, B.F. Goodrich)
   Purified water: remainder

### (Preparation Method)

Phase A and phase C are each uniformly dissolved, and then phase A is added to phase C for solubilization. After then adding phase B, the mixture is packed into a container.

### (Example 35) Pack

### (Ingredients) (wt%)

(Phase A)
   Dipropylene glycol: 5.0
   Polyoxyethylene(60 mol) hydrogenated castor oil: 5.0
(Phase B)
   Linden extract: 0.01
   Olive oil: 5.0
   Tocopherol acetate: 0.2
   Ethylparaben: 0.2
   Aromatic: 0.2
(Phase C)
   Sodium hydrogen sulfite: 0.03
   Polyvinyl alcohol (saponification degree: 90, polymerization degree: 2000): 13.0
   Ethanol: 7.0
   Purified water: remainder

### (Preparation Method)

Phase A, phase B and phase C are each uniformly dissolved, and phase B is added to phase A for solubilization. After adding this to phase C, the mixture is packed into a container.

### (Example 36) Solid foundation

### (Ingredients) (wt%)

(1) Talc: 43.1
(2) Kaolin: 15.0
(3) Sericite: 10.0
(4) Zinc flower: 7.0
(5) Titanium dioxide: 3.8
(6) Yellow iron oxide: 2.9
(7) Black iron oxide: 0.2
(8) Squalane: 8.0
(9) Isostearic acid: 4.0
(10) POE sorbitan monooleate: 3.0
(11) Isocetyl octanoate: 2.0
(12) Linden extract: 1.0
(13) Preservative: q.s.
(14) Aromatic: q.s.

### (Preparation Method)

The powder constituents of (1)-(7) are thoroughly mixed with a blender, the oily components (8)-(11) and components (12), (13) and (14) are added and thoroughly kneaded therewith, and the mixture is then packed into a container and shaped.

### (Example 37) Emulsified foundation (cream type)

### (Ingredients) (wt%)

(Powder portion)
   Titanium dioxide: 10.3
   Sericite: 5.4
   Kaolin: 3.0
   Yellow iron oxide: 0.8
   Red iron oxide: 0.3
   Black iron oxide: 0.2
(Oily phase)
   Decamethylcyclopentasiloxane: 11.5
   Liquid paraffin: 4.5
   Polyoxyethylene-modified dimethylpolysiloxane: 4.0
(Aqueous phase)
   Purified water: 51.0
   1,3-Butylene glycol: 4.5
   Linden extract: 1.5
   Sorbitan sesquioleate: 3.0
   Preservative: q.s.
   Aromatic: q.s.

### (Preparation Method)

After heating and stirring the aqueous phase, the thoroughly mixed and pulverized powder portion is added and the mixture is treated in a homomixer. The heated and mixed oily phase is added and treated in a homomixer, after which the aromatic is added while stirring and the mixture is cooled to room temperature.

### (Example 38) Cream

### (Ingredients) (wt%)

(1) Stearic acid: 5.0
(2) Stearyl alcohol: 4.0
(3) Isopropyl myristate: 18.0
(4) Glycerin monostearate: 3.0
(5) Propylene glycol: 10.0
(6) Linden extract: 0.01
(7) Caustic potash: 0.2
(8) Sodium hydrogensulfite: 0.01
(9) Preservative: q.s.
(10) Aromatic: q.s.
(11) Ion-exchanged water: remainder

### (Preparation Method)

Components (5)-(7) are added to and dissolved in (11), and the mixture is heated and held at 70°C (aqueous phase). Separately, (1)-(4) and (8)-(10) are combined, heated to melting and held at 70°C (oily phase). The oily phase is slowly added to the aqueous phase, and reaction is conducted a short while after completing the addition, while maintaining the temperature. The mixture is then homogeneously emulsified in a homomixer and cooled to 30°C while stirring.

### (Example 39) Cream

### (Ingredients) (wt%)

(1) Stearic acid: 2.0
(2) Stearyl alcohol: 7.0
(3) Hydrogenated lanolin: 2.0
(4) Squalane: 5.0
(5) 2-Octyldodecyl alcohol: 6.0
(6) Polyoxyethylene(25 mol) cetyl alcohol ether: 3.0
(7) Glycerin monostearate: 2.0
(8) Propylene glycol: 5.0
(9) Linden extract: 0.05
(10) Sodium hydrogensulfite: 0.03
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is added to the aqueous phase and pre-emulsified, and after homogeneously emulsifying in a homomixer, the mixture is cooled to 30°C while mixing.

### (Example 40) Cream

### (Ingredients) (wt%)

(1) Solid paraffin: 5.0
(2) Beeswax: 10.0
(3) Vaseline: 15.0
(4) Liquid paraffin: 41.0
(5) Glycerin monostearate: 2.0
(6) Polyoxyethylene(20 mol) sorbitan monolaurate: 2.0
(7) Soap powder: 0.1
(8) Borax: 0.2
(9) Linden extract: 0.05
(10) Sodium hydrogensulfite: 0.03
(11) Ethylparaben: 0.3
(12) Aromatic: q.s.
(13) Ion-exchanged water: remainder

### (Preparation Method)

Components (7) and (8) are added to (13), and the mixture is heated and held at 70°C (aqueous phase). Separately, (1)-(6) and (9)-(13) are combined, heated to melting and held at 70°C (oily phase). The oily phase is slowly added to the aqueous phase while stirring, for reaction. Upon completion of the reaction, the mixture is homogeneously emulsified in a homomixer, and then thoroughly stirred while cooling to 30°C.

### (Example 41) Emulsion

### (Ingredients) (wt%)

(1) Stearic acid: 2.5
(2) Cetyl alcohol: 1.5
(3) Vaseline: 5.0
(4) Liquid paraffin: 10.0
(5) Polyoxyethylene(10 mol) monooleate: 2.0
(6) Polyethylene glycol 1500: 3.0
(7) Triethanolamine: 1.0
(8) Carboxyvinyl polymer: 0.05
   (Carbopol 941, B.F. Goodrich)
(9) Linden extract: 0.01
(10) Sodium hydrogensulfite: 0.01
(11) Ethylparaben: 0.3
(12) Aromatic: q.s.
(13) Ion-exchanged water: remainder

### (Preparation Method)

Component (8) is dissolved in a small amount of (13) (phase A). Components (6) and (7) are added to and dissolved in the remainder of (13), and the mixture is heated and held at 70°C (aqueous phase). Separately, (1)-(5) and (9)-(12) are combined, heated to melting and held at 70°C (oily phase). The oily phase is added to the aqueous phase and pre-emulsified, and after adding phase A and homogeneously emulsifying in a homomixer, the mixture is cooled to 30°C while mixing.

### (Example 42) Emulsion

### (Ingredients) (wt%)

(1) Microcrystalline wax: 1.0
(2) Beeswax: 2.0
(3) Lanolin: 20.0
(4) Liquid paraffin: 10.0
(5) Squalane: 5.0
(6) Sorbitan sesquioleate: 4.0
(7) Polyoxyethylene(20 mol) sorbitan monooleate: 1.0
(8) Propylene glycol: 7.0
(9) Linden extract: 1.0
(10) Sodium hydrogensulfite: 0.01
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is stirred while the aqueous phase is slowly added thereto, and the mixture is homogeneously emulsified in a homomixer. The emulsion is then cooled to 30°C while stirring.

### (Example 43) Jelly

### (Ingredients) (wt%)

(1) 95% Ethyl alcohol: 10.0
(2) Dipropylene glycol: 15.0
(3) Polyoxyethylene(50 mol) oleyl alcohol ether: 2.0
(4) Carboxyvinyl polymer: 1.0
   (Carbopol 940, B.F. Goodrich)
(5) Caustic soda: 0.15
(6) L-Arginine: 0.1
(7) Linden extract: 7.0
(8) Sodium 2-hydroxy-4-methoxybenzophenonesulfonate: 0.05
(9) Ethylenediaminetetraacetate·3Na·2H₂O: 0.05
(10) Methylparaben: 0.2
(11) Aromatic: q.s.
(12) Ion-exchanged water: remainder

### (Preparation Method)

Component (4) is uniformly dissolved in (12) (aqueous phase). Components (7) and (3) are dissolved in (1), and this is added to the aqueous phase. After then adding (2) and (8)-(11) thereto, the mixture is neutralized and thickened with (5) and (6).

### (Example 44) Essence

### (Ingredients) (wt%)

(Phase A)
   Ethyl alcohol (95%): 10.0
   Polyoxyethylene(20 mol) octyldodecanol: 1.0
   Pantothenyl ethyl ether: 0.1
   Linden extract: 1.5
   Methylparaben: 0.15
(Phase B)
   Potassium hydroxide: 0.1
(Phase C)
   Glycerin: 5.0
   Dipropylene glycol: 10.0
   Sodium hydrogen sulfite: 0.03
   Carboxyvinyl polymer: 0.2
   (Carbopol 940, B.F. Goodrich)
   Purified water: remainder

### (Preparation Method)

Phase A and phase C are each uniformly dissolved, and then phase A is added to phase C for solubilization. After then adding phase B, the mixture is packed into a container.

### (Example 45) Pack

### (Ingredients) (wt%)

(Phase A)
   Dipropylene glycol: 5.0
   Polyoxyethylene(60 mol) hydrogenated castor oil: 5.0
(Phase B)
   Linden extract: 0.01
   Olive oil: 5.0
   Tocopherol acetate: 0.2
   Ethylparaben: 0.2
   Aromatic: 0.2
(Phase C)
   Sodium hydrogen sulfite: 0.03
   Polyvinyl alcohol (saponification degree: 90, polymerization degree: 2000): 13.0
   Ethanol: 7.0
   Purified water: remainder

### (Preparation Method)

Phase A, phase B and phase C are each uniformly dissolved, and phase B is added to phase A for solubilization. After adding this to phase C, the mixture is packed into a container.

### (Example 46) Solid foundation

### (Ingredients) (wt%)

(1) Talc: 43.1
(2) Kaolin: 15.0
(3) Sericite: 10.0
(4) Zinc flower: 7.0
(5) Titanium dioxide: 3.8
(6) Yellow iron oxide: 2.9
(7) Black iron oxide: 0.2
(8) Squalane: 8.0
(9) Isostearic acid: 4.0
(10) POE sorbitan monooleate: 3.0
(11) Isocetyl octanoate: 2.0
(12) Linden extract: 1.0
(13) Preservative: q.s.
(14) Aromatic: q.s.

### (Preparation Method)

The powder constituents of (1)-(7) are thoroughly mixed in a blender, the oily components (8)-(11) and components (12), (13) and (14) are added and thoroughly kneaded therewith, and the mixture is then packed into a container and shaped.

### (Example 47) Emulsified foundation (cream type)

### (Ingredients) (wt%)

(Powder portion)
   Titanium dioxide: 10.3
   Sericite: 5.4
   Kaolin: 3.0
   Yellow iron oxide: 0.8
   Red iron oxide: 0.3
   Black iron oxide: 0.2
(Oily phase)
   Decamethylcyclopentasiloxane: 11.5
   Liquid paraffin: 4.5
   Polyoxyethylene-modified dimethylpolysiloxane: 4.0
(Aqueous phase)
   Purified water: 51.0
   1,3-Butylene glycol: 4.5
   Linden extract: 1.5
   Sorbitan sesquioleate: 3.0
   Preservative: q.s.
   Aromatic: q.s.

### (Preparation Method)

After heating and stirring the aqueous phase, the thoroughly mixed and pulverized powder portion is added and the mixture is treated in a homomixer. The heated and mixed oily phase is added and the mixture is treated in a homomixer, after which the aromatic is added while stirring and the mixture is cooled to room temperature.

### (Example 48) Injection

Linden extract: 100 mg
Maltosyl-β-cyclodextrin: 726 mg
Sodium hydroxide: 33.3 µg
Distilled water for injection: To 5.0 ml

### (Preparation Method)

The 100 mg of linden extract is added to distilled water for injection dissolving the 726 mg of maltosyl-β-cyclodextrin. An aqueous solution of sodium hydroxide (33.3 µg) is then added to a total volume of 5 ml, and the mixture is filtered. After packing the mixture into a vial, it is freeze-dried to obtain an injection.

### (Example 49) Tablets

Linden extract: 1 g
Polyethylene glycol 6000: 10 g
Sodium lauryl sulfate: 1.5 g
Corn starch: 3 g
Lactose: 25 g
Magnesium stearate: 0.5 g

The components listed above are weighed out. Polyethylene glycol 6000 is heated to 70-80°C, and after adding linden extract, sodium lauryl sulfate, corn starch and lactose, the mixture is cooled. The solidified mixture is granulated with a grinder to obtain granules. After mixing the granules with magnesium stearate, they are compacted and tableted into 250 mg tablets.

### (Example 50) Tablets

Linden extract: 3 g
Lactose: 55 g
Potato starch: 12 g
Polyvinyl alcohol: 1.5 g
Magnesium stearate: 1.5 g

### (Preparation Method)

The components listed above are weighed out. The linden extract, lactose and potato starch are uniformly mixed. An aqueous solution of polyvinyl alcohol is added to the mixture, and granules are prepared by wet granulation. After drying the granules and mixing with magnesium stearate, they are compacted and tableted into 200 mg tablets.

### (Example 51) Capsules

Linden extract: 1 g
Lactose: 25 g
Corn starch: 5 g
Microcrystalline cellulose: 9.5 g
Magnesium stearate: 0.5 g

### (Preparation Method)

The components listed above are weighed out. The four components other than magnesium stearate are uniformly mixed. After adding magnesium stearate, mixing is continued for several minutes. The mixture is packed into 200 mg hard capsules.

### (Example 52) Powder

Linden extract: 2 g
Lactose: 79 g
Magnesium stearate: 1 g

### (Preparation Method)

The components listed above are weighed out. All of the components are uniformly mixed to form a powder.

### (Example 53) Suppository

Linden extract: 1 g
Polyethylene glycol 1500: 18 g
Polyethylene glycol 4000: 72 g

### (Preparation Method)

A 1 g rectal suppository is prepared by a melting method.

### (Example 54) Injection

Linden extract: 0.1 g
Sodium chloride: 0.9 g
Sodium hydroxide: q.s.
Water for injection: 100 mL

### (Preparation Method)

The components listed above are weighed out. The three components are dissolved in the water for injection, filtered and sterilized and then dispensed at 5 mL into a 10 mL ampule and heat sealed as an injection.

### (Example 55) Cream

### (Ingredients) (wt%)

(1) Stearic acid: 5.0
(2) Stearyl alcohol: 4.0
(3) Isopropyl myristate: 18.0
(4) Glycerin monostearate: 3.0
(5) Propylene glycol: 10.0
(6) Coix extract: 0.01
(7) Caustic potash: 0.2
(8) Sodium hydrogensulfite: 0.01
(9) Preservative: q.s.
(10) Aromatic: q.s.
(11) Ion-exchanged water: remainder

### (Preparation Method)

Components (5)-(7) are added to and dissolved in (11), and the mixture is heated and held at 70°C (aqueous phase). Separately, (1)-(4) and (8)-(10) are combined, heated to melting and held at 70°C (oily phase). The oily phase is slowly added to the aqueous phase, and reaction is conducted a short while after completing the addition, while maintaining the temperature. The mixture is then homogeneously emulsified in a homomixer and cooled to 30°C while stirring.

### (Example 56) Cream

### (Ingredients) (wt%)

(1) Stearic acid: 2.0
(2) Stearyl alcohol: 7.0
(3) Hydrogenated lanolin: 2.0
(4) Squalane: 5.0
(5) 2-Octyldodecyl alcohol: 6.0
(6) Polyoxyethylene(25 mol) cetyl alcohol ether: 3.0
(7) Glycerin monostearate: 2.0
(8) Propylene glycol: 5.0
(9) Chlorella extract: 0.05
(10) Sodium hydrogensulfite: 0.03
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is added to the aqueous phase and pre-emulsified, and after homogeneously emulsifying in a homomixer, the mixture is cooled to 30°C while mixing.

### (Example 57) Cream

### (Ingredients) (wt%)

(1) Solid paraffin: 5.0
(2) Beeswax: 10.0
(3) Vaseline: 15.0
(4) Liquid paraffin: 41.0
(5) Glycerin monostearate: 2.0
(6) Polyoxyethylene(20 mol) sorbitan monolaurate: 2.0
(7) Soap powder: 0.1
(8) Borax: 0.2
(9) Tormentilla extract: 0.05
(10) Sodium hydrogensulfite: 0.03
(11) Ethylparaben: 0.3
(12) Aromatic: q.s.
(13) Ion-exchanged water: remainder

### (Preparation Method)

Components (7) and (8) are added to (13), and the mixture is heated and held at 70°C (aqueous phase). Separately, (1)-(6) and (9)-(13) are combined, heated to melting and held at 70°C (oily phase). The oily phase is slowly added to the aqueous phase while stirring, for reaction. Upon completion of the reaction, the mixture is homogeneously emulsified in a homomixer, and then thoroughly stirred while cooling to 30°C.

### (Example 58) Emulsion

### (Ingredients) (wt%)

(1) Stearic acid: 2.5
(2) Cetyl alcohol: 1.5
(3) Vaseline: 5.0
(4) Liquid paraffin: 10.0
(5) Polyoxyethylene(10 mol) monooleate: 2.0
(6) Polyethylene glycol 1500: 3.0
(7) Triethanolamine: 1.0
(8) Carboxyvinyl polymer: 0.05
   (Carbopol 941, B.F. Goodrich)
(9) Coix extract: 0.01
(10) Chlorella extract: 0.01
(11) Sodium hydrogensulfite: 0.01
(12) Ethylparaben: 0.3
(13) Aromatic: q.s.
(14) Ion-exchanged water: remainder

### (Preparation Method)

Component (8) is dissolved in a small amount of (14) (phase A). Components (6) and (7) are added to and dissolved in the remainder of (14), and the mixture is heated and held at 70°C (aqueous phase). Separately, (1)-(5) and (9)-(14) are combined, heated to melting and held at 70°C (oily phase). The oily phase is added to the aqueous phase and pre-emulsified, and after adding phase A and homogeneously emulsifying in a homomixer, the mixture is cooled to 30°C while mixing.

### (Example 59) Emulsion

### (Ingredients) (wt%)

(1) Microcrystalline wax: 1.0
(2) Beeswax: 2.0
(3) Lanolin: 20.0
(4) Liquid paraffin: 10.0
(5) Squalane: 5.0
(6) Sorbitan sesquioleate: 4.0
(7) Polyoxyethylene(20 mol) sorbitan monooleate: 1.0
(8) Propylene glycol: 7.0
(9) Tormentilla extract: 1.0
(10) Sodium hydrogensulfite: 0.01
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is stirred while the aqueous phase is slowly added thereto, and the mixture is homogeneously emulsified in a homomixer. The emulsion is then cooled to 30°C while stirring.

### (Example 60) Jelly

### (Ingredients) (wt%)

(1) 95% Ethyl alcohol: 10.0
(2) Dipropylene glycol: 15.0
(3) Polyoxyethylene(50 mol) oleyl alcohol ether: 2.0
(4) Carboxyvinyl polymer: 1.0
   (Carbopol 940, B.F. Goodrich)
(5) Caustic soda: 0.15
(6) L-Arginine: 0.1
(7) Coix extract: 7.0
(8) Sodium 2-hydroxy-4-methoxybenzophenonesulfonate: 0.05
(9) Ethylenediaminetetraacetate·3Na·2H₂O: 0.05
(10) Methylparaben: 0.2
(11) Aromatic: q.s.
(12) Ion-exchanged water: remainder

### (Preparation Method)

Component (4) is uniformly dissolved in (12) (aqueous phase). Components (7) and (3) are dissolved in (1), and this is added to the aqueous phase. After then adding (2) and (8)-(11) thereto, the mixture is neutralized and thickened with (5) and (6).

### (Example 61) Essence

### (Ingredients) (wt%)

(Phase A)
   Ethyl alcohol (95%): 10.0
   Polyoxyethylene(20 mol) octyldodecanol: 1.0
   Pantothenyl ethyl ether: 0.1
   Chlorella extract: 1.5
   Methylparaben: 0.15
(Phase B)
   Potassium hydroxide: 0.1
(Phase C)
   Glycerin: 5.0
   Dipropylene glycol: 10.0
   Sodium hydrogen sulfite: 0.03
   Carboxyvinyl polymer: 0.2
   (Carbopol 940, B.F. Goodrich)
   Purified water: remainder

### (Preparation Method)

Phase A and phase C are each uniformly dissolved, and then phase A is added to phase C for solubilization. After then adding phase B, the mixture is packed into a container.

### (Example 62) Pack

### (Ingredients) (wt%)

(Phase A)
   Dipropylene glycol: 5.0
   Polyoxyethylene(60 mol) hydrogenated castor oil: 5.0
(Phase B)
   Tormentilla extract: 0.01
   Olive oil: 5.0
   Tocopherol acetate: 0.2
   Ethylparaben: 0.2
   Aromatic: 0.2
(Phase C)
   Sodium hydrogen sulfite: 0.03
   Polyvinyl alcohol (saponification degree: 90, polymerization degree: 2000): 13.0
   Ethanol: 7.0
   Purified water: remainder

### (Preparation Method)

Phase A, phase B and phase C are each uniformly dissolved, and phase B is added to phase A for solubilization. After adding this to phase C, the mixture is packed into a container.

### (Example 63) Solid foundation

### (Ingredients) (wt%)

(1) Talc: 43.1
(2) Kaolin: 15.0
(3) Sericite: 10.0
(4) Zinc flower: 7.0
(5) Titanium dioxide: 3.8
(6) Yellow iron oxide: 2.9
(7) Black iron oxide: 0.2
(8) Squalane: 8.0
(9) Isostearic acid: 4.0
(10) POE sorbitan monooleate: 3.0
(11) Isocetyl octanoate: 2.0
(12) Coix extract: 1.0
(13) Preservative: q.s.
(14) Aromatic: q.s.

### (Preparation Method)

The powder constituents of (1)-(7) are thoroughly mixed in a blender, the oily components (8)-(11) and components (12), (13) and (14) are added and thoroughly kneaded therewith, and the mixture is then packed into a container and shaped.

### (Example 64) Emulsified foundation (cream type)

### (Ingredients) (wt%)

(Powder portion)
   Titanium dioxide: 10.3
   Sericite: 5.4
   Kaolin: 3.0
   Yellow iron oxide: 0.8
   Red iron oxide: 0.3
   Black iron oxide: 0.2
(Oily phase)
   Decamethylcyclopentasiloxane: 11.5
   Liquid paraffin: 4.5
   Polyoxyethylene-modified dimethylpolysiloxane: 4.0
(Aqueous phase)
   Purified water: 51.0
   1,3-Butylene glycol: 4.5
   Chlorella extract: 1.5
   Sorbitan sesquioleate: 3.0
   Preservative: q.s.
   Aromatic: q.s.

### (Preparation Method)

After heating and stirring the aqueous phase, the thoroughly mixed and pulverized powder portion is added and the mixture is treated in a homomixer. The heated and mixed oily phase is added and treated in a homomixer, after which the aromatic is added while stirring and the mixture is cooled to room temperature.

### (Example 65) Cream

### (Ingredients) (wt%)

(1) Stearic acid: 5.0
(2) Stearyl alcohol: 4.0
(3) Isopropyl myristate: 18.0
(4) Glycerin monostearate: 3.0
(5) Propylene glycol: 10.0
(6) Tormentilla extract: 0.01
(7) Caustic potash: 0.2
(8) Sodium hydrogensulfite: 0.01
(9) Preservative: q.s.
(10) Aromatic: q.s.
(11) Ion-exchanged water: remainder

### (Preparation Method)

Components (5)-(7) are added to and dissolved in (11), and the mixture is heated and held at 70°C (aqueous phase). Separately, (1)-(4) and (8)-(10) are combined, heated to melting and held at 70°C (oily phase). The oily phase is slowly added to the aqueous phase, and reaction is conducted a short while after completing the addition, while maintaining the temperature. The mixture is then homogeneously emulsified in a homomixer and cooled to 30°C while stirring.

### (Example 66) Cream

### (Ingredients) (wt%)

(1) Stearic acid: 2.0
(2) Stearyl alcohol: 7.0
(3) Hydrogenated lanolin: 2.0
(4) Squalane: 5.0
(5) 2-Octyldodecyl alcohol: 6.0
(6) Polyoxyethylene(25 mol) cetyl alcohol ether: 3.0
(7) Glycerin monostearate: 2.0
(8) Propylene glycol: 5.0
(9) Coix extract: 0.05
(10) Sodium hydrogensulfite: 0.03
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is added to the aqueous phase and pre-emulsified, and after homogeneously emulsifying in a homomixer, the mixture is cooled to 30°C while mixing.

### (Example 67) Cream

### (Ingredients) (wt%)

(1) Solid paraffin: 5.0
(2) Beeswax: 10.0
(3) Vaseline: 15.0
(4) Liquid paraffin: 41.0
(5) Glycerin monostearate: 2.0
(6) Polyoxyethylene(20 mol) sorbitan monolaurate: 2.0
(7) Soap powder: 0.1
(8) Borax: 0.2
(9) Chlorella extract: 0.05
(10) Sodium hydrogensulfite: 0.03
(11) Ethylparaben: 0.3
(12) Aromatic: q.s.
(13) Ion-exchanged water: remainder

### (Preparation Method)

Components (7) and (8) are added to (13), and the mixture is heated and held at 70°C (aqueous phase). Separately, (1)-(6) and (9)-(13) are combined, heated to melting and held at 70°C (oily phase). The oily phase is slowly added to the aqueous phase while stirring, for reaction. Upon completion of the reaction, the mixture is homogeneously emulsified in a homomixer, and then thoroughly stirred while cooling to 30°C.

### (Example 68) Emulsion

### (Ingredients) (wt%)

(1) Stearic acid: 2.5
(2) Cetyl alcohol: 1.5
(3) Vaseline: 5.0
(4) Liquid paraffin: 10.0
(5) Polyoxyethylene(10 mol) monooleate: 2.0
(6) Polyethylene glycol 1500: 3.0
(7) Triethanolamine: 1.0
(8) Carboxyvinyl polymer: 0.05
   (Carbopol 941, B.F. Goodrich)
(9) Tormentilla extract: 0.01
(10) Sodium hydrogensulfite: 0.01
(11) Ethylparaben: 0.3
(12) Aromatic: q.s.
(13) Ion-exchanged water: remainder

### (Preparation Method)

Component (8) is dissolved in a small amount of (13) (phase A). Components (6) and (7) are added to and dissolved in the remainder of (13), and the mixture is heated and held at 70°C (aqueous phase). Separately, (1)-(5) and (9)-(12) are combined, heated to melting and held at 70°C (oily phase). The oily phase is added to the aqueous phase and pre-emulsified, and after adding phase A and homogeneously emulsifying in a homomixer, the mixture is cooled to 30°C while mixing.

### (Example 69) Emulsion

### (Ingredients) (wt%)

(1) Microcrystalline wax: 1.0
(2) Beeswax: 2.0
(3) Lanolin: 20.0
(4) Liquid paraffin: 10.0
(5) Squalane: 5.0
(6) Sorbitan sesquioleate: 4.0
(7) Polyoxyethylene(20 mol) sorbitan monooleate: 1.0
(8) Propylene glycol: 7.0
(9) Coix extract: 1.0
(10) Sodium hydrogensulfite: 0.01
(11) Ethylparaben: 0.3
(12) Aromatic: q.s.
(13) Ion-exchanged water: remainder

### (Preparation Method)

Component (8) is added to (13), and the mixture is heated and held at 70°C (aqueous phase). Separately, (1)-(7) and (9)-(12) are combined, heated to melting and held at 70°C (oily phase). The oily phase is stirred while the aqueous phase is slowly added thereto, and the mixture is homogeneously emulsified in a homomixer. The emulsion is then cooled to 30°C while stirring.

### (Example 70) Jelly

### (Ingredients) (wt%)

(1) 95% Ethyl alcohol: 10.0
(2) Dipropylene glycol: 15.0
(3) Polyoxyethylene(50 mol) oleyl alcohol ether: 2.0
(4) Carboxyvinyl polymer: 1.0
   (Carbopol 940, B.F. Goodrich)
(5) Caustic soda: 0.15
(6) L-Arginine: 0.1
(7) Chlorella extract: 7.0
(8) Sodium 2-hydroxy-4-methoxybenzophenonesulfonate: 0.05
(9) Ethylenediaminetetraacetate·3Na·2H₂O: 0.05
(10) Methylparaben: 0.2
(11) Aromatic: q.s.
(12) Ion-exchanged water: remainder

### (Preparation Method)

Component (4) is uniformly dissolved in (12) (aqueous phase). Components (7) and (3) are dissolved in (1), and this is added to the aqueous phase. After then adding (2) and (8)-(11) thereto, the mixture is neutralized and thickened with (5) and (6).

### (Example 71) Essence

### (Ingredients) (wt%)

(Phase A)
   Ethyl alcohol (95%): 10.0
   Polyoxyethylene(20 mol) octyldodecanol: 1.0
   Pantothenyl ethyl ether: 0.1
   Tormentilla extract: 1.5
   Methylparaben: 0.15
(Phase B)
   Potassium hydroxide: 0.1
(Phase C)
   Glycerin: 5.0
   Dipropylene glycol: 10.0
   Sodium hydrogen sulfite: 0.03
   Carboxyvinyl polymer: 0.2
   (Carbopol 940, B.F. Goodrich)
   Purified water: remainder

### (Preparation Method)

Phase A and phase C are each uniformly dissolved, and then phase A is added to phase C for solubilization. After then adding phase B, the mixture is packed into a container.

### (Example 72) Pack

### (Ingredients) (wt%)

(Phase A)
   Dipropylene glycol: 5.0
   Polyoxyethylene(60 mol) hydrogenated castor oil: 5.0
(Phase B)
   Coix extract: 0.01
   Olive oil: 5.0
   Tocopherol acetate: 0.2
   Ethylparaben: 0.2
   Aromatic: 0.2
(Phase C)
   Sodium hydrogen sulfite: 0.03
   Polyvinyl alcohol (saponification degree: 90,
   polymerization degree: 2000): 13.0
   Ethanol: 7.0
   Purified water: remainder

### (Preparation Method)

Phase A, phase B and phase C are each uniformly dissolved, and phase B is added to phase A for solubilization. After adding this to phase C, the mixture is packed into a container.

### (Example 73) Solid foundation

### (Ingredients) (wt%)

(1) Talc: 43.1
(2) Kaolin: 15.0
(3) Sericite: 10.0
(4) Zinc flower: 7.0
(5) Titanium dioxide: 3.8
(6) Yellow iron oxide: 2.9
(7) Black iron oxide: 0.2
(8) Squalane: 8.0
(9) Isostearic acid: 4.0
(10) POE sorbitan monooleate: 3.0
(11) Isocetyl octanoate: 2.0
(12) Chlorella extract: 1.0
(13) Preservative: q.s.
(14) Aromatic: q.s.

### (Preparation Method)

The powder constituents of (1)-(7) are thoroughly mixed in a blender, the oily components (8)-(11) and components (12), (13) and (14) are added and thoroughly kneaded therewith, and the mixture is then packed into a container and shaped.

### (Example 74) Emulsified foundation (cream type)

### (Ingredients) (wt%)

(Powder portion)
   Titanium dioxide: 10.3
   Sericite: 5.4
   Kaolin: 3.0
   Yellow iron oxide: 0.8
   Red iron oxide: 0.3
   Black iron oxide: 0.2
(Oily phase)
   Decamethylcyclopentasiloxane: 11.5
   Liquid paraffin: 4.5
   Polyoxyethylene-modified dimethylpolysiloxane: 4.0
(Aqueous phase)
   Purified water: 51.0
   1,3-Butylene glycol: 4.5
   Tormentilla extract: 1.5
   Sorbitan sesquioleate: 3.0
   Preservative: q.s.
   Aromatic: q.s.

### (Preparation Method)

After heating and stirring the aqueous phase, the thoroughly mixed and pulverized powder portion is added and the mixture is treated in a homomixer. The heated and mixed oily phase is added and the resulting mixture is treated in a homomixer, after which the aromatic is added while stirring and the mixture is cooled to room temperature.

### (Example 75) Injection

Coix extract: 100 mg
Maltosyl-β-cyclodextrin: 726 mg
Sodium hydroxide: 33.3 µg
Distilled water for injection: To 5.0 ml

### (Preparation Method)

The 100 mg of coix extract is added to distilled water for injection dissolving the 726 mg of maltosyl-β-cyclodextrin. An aqueous solution of sodium hydroxide (33.3 µg) is then added to a total volume of 5 ml, and the mixture is filtered. After packing the mixture into a vial, it is freeze-dried to obtain an injection.

### (Example 76) Tablets

Chlorella extract: 1 g
Polyethylene glycol 6000: 10 g
Sodium lauryl sulfate: 1.5 g
Corn starch: 3 g
Lactose: 25 g
Magnesium stearate: 0.5 g

### (Preparation Method)

The components listed above are weighed out. Polyethylene glycol 6000 is heated to 70-80°C, and after adding chlorella extract, sodium lauryl sulfate, corn starch and lactose, the mixture is cooled. The solidified mixture is granulated with a grinder to obtain granules. After mixing the granules with magnesium stearate, they are compacted and tableted into 250 mg tablets.

### (Example 77) Tablets

Tormentilla extract: 3 g
Lactose: 55 g
Potato starch: 12 g
Polyvinyl alcohol: 1.5 g
Magnesium stearate: 1.5 g

### (Preparation Method)

The components listed above are weighed out. The tormentilla extract, lactose and potato starch are uniformly mixed. An aqueous solution of polyvinyl alcohol is added to the mixture, and granules are prepared by wet granulation. After drying the granules and mixing with magnesium stearate, they are compacted and tableted into 200 mg tablets.

### (Example 78) Capsules

Coix extract: 1 g
Lactose: 25 g
Corn starch: 5 g
Microcrystalline cellulose: 9.5 g
Magnesium stearate: 0.5 g

### (Preparation Method)

The components listed above are weighed out. The four components other than magnesium stearate are uniformly mixed. After adding magnesium stearate, mixing is continued for several minutes. The mixture is packed into 200 mg hard capsules.

### (Example 79) Powder

Chlorella extract: 2 g
Lactose: 79 g
Magnesium stearate: 1 g

### (Preparation Method)

The components listed above are weighed out. All of the components are uniformly mixed to form a powder.

### (Example 80) Suppository

Tormentilla extract: 1 g
Polyethylene glycol 1500: 18 g
Polyethylene glycol 4000: 72 g

### (Preparation Method)

A 1 g rectal suppository is prepared by a melting method.

### (Example 81) Injection

Tormentilla extract: 0.1 g
Sodium chloride: 0.9 g
Sodium hydroxide: q.s.
Water for injection: 100 mL

### (Preparation Method)

The components listed above are weighed out. The three components are dissolved in the water for injection, filtered and sterilized and then dispensed at 5 mL into a 10 mL ampule and heat sealed as an injection.

## Claims

1. An anti-aging agent comprising one or more galenicals selected from the group consisting of mukurossi peel extract and hazelnut extract.

2. An angiogenesis inhibitor comprising one or more galenicals selected from the group consisting of mukurossi peel extract and hazelnut extract.

3. A vascular endothelial growth factor (VEGF) expression inhibitor comprising one or more galenicals selected from the group consisting of mukurossi peel extract and hazelnut extract.

4. A cosmetic method that inhibits aging, the method including application of an anti-aging agent according to claim 1 to skin.

5. An angiogenesis inhibitor **characterized by** comprising linden extract as an active component.

6. A vascular endothelial growth factor (VEGF) expression inhibitor **characterized by** comprising linden extract as an active component.

7. A vascular endothelial growth factor (VEGF) expression inhibitor **characterized by** comprising one or more galenical extracts selected from the group consisting of coix extract, chlorella extract and tormentilla extract, as active components.
